# DEMANDE DE BREVET EUROPEEN

(11) **EP 3 666 879 A1**
(43) Date de publication de la demande: **17.06.2020**
(21) Numéro de dépôt: 19214664.5
(22) Date de dépôt: 10.12.2019
(51) Int. Cl.: C12M 1/00

(54) **PHOTOBIOREACTEUR**

(30) Priorité: 10.12.2018 FR 1872612
(71) Demandeur: Commissariat à l'Energie Atomique et aux Energies Alternatives, 75015 Paris (FR)
(72) Inventeur: DUCROS, Cédric, 38054 Grenoble Cedex 09 (FR)
(74) Mandataire: Nony

(57) **Abrégé**

Photobioréacteur (5) pour cultiver un microorganisme photosynthétique (55), comportant :
- une enceinte (10) comportant une paroi de captation de lumière (70) et définissant une chambre de culture (45) pour contenir un milieu de culture (50) contenant au moins le microorganisme photosynthétique, la paroi de captation de lumière étant transparente à un rayonnement dans l'infrarouge et à un rayonnement dans le visible, et
- un filtre optique (15) pour filtrer un rayonnement d'irradiation dirigé vers la chambre de culture,
le filtre optique étant transparent au rayonnement dans le visible et contenant un composé thermochrome,
le filtre optique étant transparent au rayonnement dans l'infrarouge à une température d'au moins 10°C inférieure à la température de transition du composé thermochrome et présentant une transmittance optique au rayonnement dans l'infrarouge inférieure ou égale à 20 % à une température d'au moins 10°C supérieure à la température de transition du composé thermochrome.

## Description

### Domaine technique

La présente invention concerne le domaine des photobioréacteurs adaptés à la culture d'un microorganisme photosynthétique.

### Technique antérieure

Un microorganisme photosynthétique est généralement cultivé au moyen d'un photobioréacteur. Par exemple, un photobioréacteur peut être intégré dans un bâtiment, de manière à favoriser les échanges thermiques entre le photobioréacteur et le bâtiment. Un photobioréacteur comporte classiquement une cuve contenant un milieu de culture comportant le microorganisme photosynthétique, la cuve étant généralement recouverte d'un couvercle transparent à la lumière et plus favorablement intégralement transparente. En variante, le photobioréacteur peut comporter une structure constituée de tubes creux et transparents au sein desquels circule le milieu de culture, en boucle ouverte ou fermée. Le photobioréacteur définit ainsi une chambre de culture qui isole le milieu de culture et le microorganisme photosynthétique de l'atmosphère environnante. La croissance du microorganisme photosynthétique est notamment assurée par l'exposition du milieu de culture à un rayonnement lumineux. Par exemple, lorsqu'intégré dans un bâtiment, le photobioréacteur est exposé au rayonnement solaire. Un tel rayonnement présente des composantes spectrales dans l'infrarouge, qui provoquent un échauffement du milieu de culture. De plus, le rayonnement solaire varie quotidiennement et saisonnièrement, ce qui induit des variations de température du milieu de culture. Or, pour que la croissance du microorganisme photosynthétique soit optimale, la température du milieu de culture doit être comprise dans une plage de températures optimales pour la croissance du microorganisme photosynthétique cultivé, généralement comprises entre 25°C et 40°C. Cependant, l'échauffement du milieu de culture peut limiter, voire bloquer la croissance du microorganisme photosynthétique, et s'il est excessif, peut en provoquer la mort. Une mortalité excessive du microorganisme photosynthétique entraîne une dégradation du rendement du photobioréacteur.

Par ailleurs, parmi le spectre du rayonnement solaire, les composantes dans le visible sont celles qui favorisent une croissance optimale du microorganisme photosynthétique.

Pour assurer un rendement élevé, la culture du microorganisme photosynthétique nécessite donc de maximiser l'exposition au rayonnement lumineux, notamment aux composantes dans le visible du rayonnement lumineux, pour favoriser la croissance du microorganisme photosynthétique, tout en assurant que la température du milieu de culture soit maintenue dans la plage de températures de croissance optimale, spécifique au microorganisme photosynthétique.

Pour réguler la température du milieu de culture, il est par exemple connu de WO 2007/129327 A1 d'asperger le photobioréacteur d'eau, voire de l'immerger entièrement dans l'eau. Toutefois, une telle régulation requiert un apport d'eau externe ainsi que la construction d'une infrastructure autour du photobioréacteur, ce qui augmente le coût de la culture du microorganisme photo synthétique. De plus, l'aspersion ou l'immersion peut conduire à un encrassement de l'enveloppe du photobioréacteur et induire des réflexions optiques parasites, ce qui réduit l'absorption du rayonnement lumineux par le microorganisme photosynthétique.

Il est aussi connu de mettre en œuvre une unité de régulation de la température du milieu de culture. A cette fin, la demande WO 2007/129327 A1 décrit un échangeur externe dans lequel circule le milieu de culture. La demande FR 2 823 761 A1 décrit un photobioréacteur comportant une enveloppe dans laquelle circule un fluide caloporteur pour échanger de la chaleur avec le milieu de culture. Dans la demande EP 0 647 707 A1, le photobioréacteur comporte une paroi dans laquelle est logée une unité de chauffage et de refroidissement pour réguler la température du milieu de culture. Les solutions techniques décrites dans les trois documents précités grèvent toutefois le coût d'exploitation du photobioréacteur.

Un photobioréacteur est encore connu du brevet EP 1 928 994 B1, qui est formé de tubes revêtus d'une couche formant barrière thermique. La demande FR 2 964 666 A1 décrit un photobioréacteur équipé d'une soupape thermique constituée d'un matériau à changement de phase, par exemple de la paraffine, de sorte à maintenir passivement la température du milieu de culture sous une température seuil. La demande FR 2 978 772 A1 décrit enfin un photobioréacteur dont une paroi est formée d'un vitrage dont une face est recouverte d'un empilement de couches minces antireflet et de couches minces fonctionnelles. L'empilement forme un filtre optique ne transmettant que les composantes du rayonnement solaire utiles à la photosynthèse. Le photobioréacteur de la demande FR 2 978 772 A1 limite ainsi le risque d'échauffement excessif du milieu de culture, notamment en période estivale, le filtre réfléchissant les composantes dans l'infrarouge du rayonnement solaire. Cependant, la filtration des composantes dans l'infrarouge peut s'avérer néfaste, notamment en période hivernale, car elle prive le milieu de culture d'un apport de chaleur bénéfique pour maintenir la température du milieu de culture à une valeur minimale pour la croissance du microorganisme photo synthétique.

Il demeure donc un besoin pour un photobioréacteur qui assure une croissance optimale d'un microorganisme photosynthétique et à moindre coût, lorsqu'il est exposé à un rayonnement lumineux variable temporellement, notamment solaire.

L'invention vise à satisfaire ce besoin et propose un photobioréacteur pour la culture d'un microorganisme photosynthétique, le photobioréacteur comportant :
- une enceinte comportant une paroi de captation de lumière et définissant une chambre de culture pour contenir un milieu de culture contenant au moins le microorganisme photosynthétique, la paroi de captation de lumière étant transparente à un rayonnement dans l'infrarouge et à un rayonnement dans le visible, et
- un filtre optique pour filtrer un rayonnement d'irradiation dirigé vers la chambre de culture, le filtre optique étant transparent au rayonnement dans le visible et contenant au moins un composé thermochrome,
le filtre optique étant transparent au rayonnement dans l'infrarouge à une température d'au moins 10°C inférieure à la température de transition du composé thermochrome et présentant une transmittance optique au rayonnement dans l'infrarouge inférieure ou égale à 20 % à une température d'au moins 10°C supérieure à la température de transition du composé thermochrome.

Le filtre optique du photobioréacteur selon l'invention favorise la transmission vers la chambre de culture d'une portion suffisante de l'intensité du spectre dans le visible du rayonnement d'irradiation pour assurer une activité photosynthétique optimale du microorganisme photosynthétique. En outre, le filtre optique régule passivement et à moindre coût la température du milieu de culture. Notamment, lorsque la température du filtre optique est inférieure à la borne minimale de la plage de températures optimales pour la croissance du microorganisme photosynthétique et est inférieure à la température de transition, par exemple en conditions hivernales et/ou en début de matinée et fin d'après-midi, la partie des composantes dans l'infrarouge du rayonnement d'irradiation transmise par le filtre optique dans la chambre de culture favorise l'élévation de température du milieu de culture. La température du milieu de culture augmente ainsi progressivement jusqu'à être comprise dans la plage de températures optimales de croissance du microorganisme photosynthétique. L'activité photosynthétique du microorganisme photosynthétique devient conséquemment plus intense. Lorsque la température du filtre optique est supérieure à la borne maximale de la plage de températures optimales pour la croissance du microorganisme photosynthétique et est supérieure à la température de transition, le filtre optique limite la transmission des composantes dans l'infrarouge du rayonnement d'irradiation vers la chambre de culture. L'échauffement du milieu de culture est ainsi limité, mieux est nul. La mort du microorganisme photosynthétique liée à une élévation excessive de la température du milieu de culture peut ainsi être évitée. La température du milieu de culture décroit alors progressivement jusqu'à être comprise dans la plage de températures optimales pour la croissance du microorganisme photo synthétique. Ainsi, l'activité photosynthétique du microorganisme photosynthétique s'intensifie.

Par « rayonnement dans le visible », on entend un rayonnement lumineux dont le spectre comporte au moins une, de préférence une pluralité de composantes dont les longueurs d'ondes respectives sont comprises entre 350 nm et 780 nm, de préférence entre 350 nm et 750 nm.

Par « rayonnement infrarouge », on entend un rayonnement lumineux dont le spectre comporte au moins une, de préférence une pluralité de composantes dont les longueurs d'ondes respectives sont supérieures à 780 nm, de préférence supérieures à 1100 nm.

Par « transmittance optique » d'un corps à un rayonnement, on entend le rapport, exprimé en pourcentage, de l'intensité du flux transmis à travers le corps sur l'intensité du flux du rayonnement incident auquel le corps est exposé. Lorsque le rayonnement lumineux est polychromatique, les intensités respectives des flux incident et transmis sont calculées par intégration, sur le spectre de longueurs d'ondes constituant le rayonnement lumineux, des flux élémentaires associées aux longueurs d'onde respectives. Au sens de l'invention, on considère la transparence optique du corps dans une plage de températures comprise entre 0°C et 70°C.

Un corps « transparent » à un rayonnement lumineux présente une transmittance optique audit rayonnement lumineux de préférence supérieure à 80 %, voire supérieure à 90%.

Le rayonnement d'irradiation peut comporter des composantes dans l'infrarouge et dans le visible. Il peut notamment être le rayonnement solaire.

De préférence, la température de transition du composé thermochrome est supérieure ou égale à 30°C, de préférence supérieure ou égale à 35°C et/ou inférieure ou égale à 55°C, et de préférence inférieure ou égale à 50°C, voire même inférieure ou égale à 45°C. Elle est par exemple comprise entre 38 °C et 42°C. On optimise ainsi le rendement du photobioréacteur, notamment lorsque l'activité photosynthétique du microorganisme photosynthétique est optimale dans une plage de température comprise entre 25°C et 40°C. De préférence, la température de transition du composé thermochrome est choisie en fonction de la nature du microorganisme photosynthétique à cultiver.

Un composé «thermochrome» est caractérisé par une variation réversible d'une propriété optique avec une variation de la température du composé. La « température de transition » caractérise un changement d'état, notamment un changement de phase, du composé thermochrome. Le changement d'état se traduit par une variation discontinue des indices optiques, *i.e.* indice de réfraction *n* et coefficient d'extinction *k,* du composé thermochrome. En particulier, le composé thermochrome peut présenter un coefficient d'extinction dans l'infrarouge au moins 3 fois supérieur au coefficient d'extinction dans le visible et un indice de réfraction dans l'infrarouge au moins 2 fois inférieur à l'indice de réfraction dans le visible. Par ailleurs, le composé thermochrome présente de préférence un coefficient d'extinction et/ou un indice de réfraction sensiblement identique dans l'infrarouge et dans le visible.

La température de transition peut être déterminée en mesurant les indices optiques du composé thermochrome.

Les mesures sont par exemple effectuées au moyen de deux ellipsomètres équipés chacun d'une cellule chauffante, de manière à chauffer le composé thermochrome à des températures comprises entre 20 °C et 100 °C. Les mesures des indices optiques dans des plages de longueurs d'onde dans le visible et dans l'infrarouge sont effectuées au moyen des premier et deuxième ellipsomètres respectivement, de manière à caractériser la relation entre les indices optiques et la longueur d'onde du rayonnement.

Le composé thermochrome peut être choisi dans le groupe formé par les cristaux liquides thermochromes, les leuco-colorants thermochromes, les oxydes thermochromes, optionnellement dopés, et leurs mélanges. Le composé thermochrome peut être un oxyde thermochrome, optionnellement dopé, choisi dans le groupe formé par VO₂, BiVO₄, NbO₂ et leurs mélanges. L'oxyde de vanadium, dopé au tungstène VO₂:W est le composé thermochrome préféré. En particulier, le taux de dopage en tungstène peut être choisi de sorte que l'oxyde de vanadium dopé au tungstène VO₂:W présente une température de transition comprise entre 35°C et 45°C, par exemple égale à 40°C. De préférence, le taux de dopage en tungstène de VO₂:W, défini comme le rapport de la masse de tungstène sur la masse d'oxyde de vanadium est compris entre 0,5 % et 1 %. L'oxyde de vanadium, dopé ou non, présente une forme cristalline pseudo-rutile, dénommée M, et est semi-conducteur en dessous de la température de transition, et présente une forme cristalline rutile, dénommée R et est métallique au-dessus de la température de transition. Les formes M et R de l'oxyde de vanadium sont différentes des formes A, B, C, M1 et M2 aussi connues de l'oxyde de vanadium.

L'oxyde de vanadium, dopé ou non, présente un contraste de résistivité électrique entre la forme pseudo-rutile et la forme rutile, qui peut être de l'ordre de 10³ Ω.cm lorsque la structure de l'oxyde est polycristalline et de l'ordre de 10⁴ Ω.cm lorsque la structure de l'oxyde est monocristalline. La variation de résistivité électrique se traduit par une variation de l'indice optique complexe, les propriétés optiques de l'oxyde étant modifiées par la libération de porteurs de charges lors du changement de phase à la température de transition.

En ce qui concerne le filtre optique, il peut être constitué intégralement par le composé thermochrome.

Le filtre optique est notamment disposé de sorte que la portion dans le visible du rayonnement d'irradiation transmise par le filtre optique et la paroi de captation de lumière accède, de préférence directement, à la chambre de culture, lorsque le photobioréacteur est exposé au rayonnement d'irradiation. Par accès « direct » à la chambre de culture, on entend que la portion dans le visible du rayonnement d'irradiation transmise par l'ensemble formé par la captation de lumière et par le filtre optique n'est pas déviée avant d'accéder à la chambre de culture.

De préférence, le filtre optique est superposé à la paroi de captation de lumière et à la chambre de culture.

De préférence, le filtre optique est au contact de la paroi de captation de lumière. De préférence, il se présente sous la forme d'un revêtement disposé sur une face de la paroi de captation de lumière, de préférence sur la face de la paroi de captation de lumière opposée à la face de ladite paroi en regard de la chambre de culture. Le revêtement peut recouvrir partiellement la face de la paroi de captation de lumière sur laquelle il est disposé. Par exemple, il peut recouvrir plus de 30 %, voire plus de 50 %, voire même plus de 80 % de l'aire de la face de la paroi de captation de lumière sur laquelle il est disposé. De préférence, il recouvre intégralement la face de la paroi de captation de lumière sur laquelle il est disposé.

Le revêtement peut présenter des faces externe et interne, la face interne étant au contact de la paroi de captation de lumière, la face externe étant opposée à la face interne. Les faces externe et interne du revêtement peuvent être parallèles.

La face externe et/ou la face interne du revêtement présentent de préférence une texture rugueuse sous la forme d'un réseau formé par une succession régulière d'un motif en relief.

Le motif en relief peut présenter une forme variée, et notamment pyramidale.

La texture rugueuse de la face externe et/ou la texture rugueuse de la face interne du revêtement limite la réflexion du rayonnement lumineux incident, notamment du rayonnement solaire qui présente une incidence variable en fonction de l'heure de la journée. La texture rugueuse de la face externe et/ou la texture rugueuse de la face interne du revêtement améliore ainsi la transmittance optique du filtre optique au rayonnement dans le visible incident, en particulier à la composante dans le visible d'un rayonnement solaire.

Dans la variante où les faces interne et externe du revêtement présentent chacune une texture rugueuse, les motifs en relief de la face externe et de la face interne du revêtement peuvent être identiques. De préférence, ils sont différents, de manière à maximiser la fonction d'antireflet procurée par chaque texture rugueuse en prenant en compte la variation d'indice optique entre les milieux que chacune des faces externe et face interne du revêtement sépare.

La texture rugueuse de la face externe du revêtement présente de préférence un pas moyen, correspondant à la distance moyenne entre deux reliefs consécutifs du motif en relief, compris entre 50 nm et 300 nm, de préférence entre 50 nm et 100 nm, et/ou une hauteur moyenne des motifs en reliefs comprise entre 100 nm et 600 nm, de préférence comprise entre 100 nm et 200 nm. Le pas moyen et la hauteur moyenne des motifs peut être mesurée sur de photographies acquises au moyen d'un microscope électronique à balayage.

Le rapport de texturation est égal au rapport de la hauteur moyenne des motifs en relief sur le pas moyen. Le rapport de texturation de la face externe est de préférence compris entre 1,3 et 2,0 de préférence compris entre 1,5 et 1,9.

La texture rugueuse de la face externe du revêtement peut être obtenue par un procédé comportant une étape de gravure sèche de la face de la paroi de captation de lumière sur laquelle le revêtement est disposé, suivie d'une étape de dépôt du revêtement sur ladite face ainsi gravée. Différentes rugosités de la texture rugueuse de la face externe du revêtement peuvent être obtenues en faisant varier les paramètres de conduite de l'étape de gravure sèche, qui seront décrits ci-après. La texture rugueuse de la face externe du revêtement peut ainsi définir une réplique de la texture de la face de la paroi de captation de lumière sur laquelle le revêtement est disposé.

Le revêtement peut être obtenu par un par un procédé de dépôt choisi parmi le dépôt chimique en phase vapeur et le dépôt physique en phase vapeur, notamment la pulvérisation cathodique magnétron.

Le photobioréacteur peut comporter deux filtres optiques se présentant chacun sous la forme d'un revêtement et étant disposés sur les deux faces opposées de la paroi de captation de lumière.

Le filtre optique peut comporter un support transparent au rayonnement dans le visible et au rayonnement dans l'infrarouge recouvert par le revêtement. Le support est par exemple en un matériau identique au matériau constitutif de la paroi de captation de lumière. Selon cette autre variante, le filtre optique est par exemple disposé à distance de l'enceinte.

Le filtre optique peut présenter une transmittance optique au rayonnement dans l'infrarouge supérieure ou égale à 80 %, voire supérieure ou égale à 90 %, respectivement inférieure ou égale à 20 %, voire inférieure ou égale à 10 %, à une température d'au moins 5°C inférieure, respectivement d'au moins 5°C supérieure à la température de transition du composé thermochrome.

De préférence, la paroi de captation de lumière comporte, pour plus de 90% de sa masse, un matériau choisi parmi un verre, de préférence un verre borosilicate ou un verre sodocalcique, et un polymère thermoplastique, de préférence un polycarbonate ou un polyméthacrylate de méthyle.

La paroi de captation de lumière peut comporter une face externe en regard de la chambre de culture et une face interne opposée à la face externe et au contact du filtre optique.

La face externe et/ou la face interne de la paroi de captation de lumière peuvent présenter une texture rugueuse présentant, de préférence, la forme d'un réseau formé par une succession régulière d'un motif en relief.

La texture rugueuse de la face externe de la paroi de captation de lumière limite la réflexion du rayonnement lumineux incident, notamment solaire qui présente une incidence variable en fonction de l'heure de la journée. Elle augmente la transmittance optique de la paroi de captation de lumière et de l'ensemble formé par la paroi de captation de la lumière et par le filtre optique, au rayonnement dans le visible incident, en particulier à la composante dans le visible d'un rayonnement solaire.

La texture rugueuse de la face externe de la paroi de captation de lumière présente de préférence un pas moyen entre deux reliefs consécutifs compris entre 40 nm et 600 nm, de préférence compris entre 80 nm et 300 nm, et/ou une hauteur moyenne des motifs en reliefs comprise entre 70 nm et 800 nm, de préférence comprise entre 100 nm et 600 nm.

Le rapport de texturation de la face interne de la paroi de captation de lumière est de préférence compris entre 1,30 et 2,00, de préférence compris entre 1,50 et 1,90, notamment de sorte à assurer une homogénéité de l'épaisseur du filtre optique, lorsque ce dernier est déposé, comme décrit ci-dessus, sous la forme d'un revêtement sur la face interne de la paroi de captation de lumière.

La texture rugueuse de la face interne de la paroi de captation de lumière peut être obtenue par un procédé de gravure sèche. En particulier, elle peut être obtenue par un procédé de gravure plasma sous vide, de préférence opéré au moyen d'un mélange gazeux de trifluorométhane CHF3 et de dioxygène O₂, le rapport molaire CHF₃/O₂ étant compris entre 10,0 et 15,0, sous une pression comprise entre 50 mTorr et 200 mTorr, avec une densité de travail comprise entre 1,65 W.cm⁻² et 3,56 W.cm⁻² et pendant une durée comprise entre 10 minutes et 30 minutes.

La présence d'une texture rugueuse améliore la transmittance optique de la paroi de captation de lumière.

En particulier, la paroi de captation de lumière peut présenter une réflexion totale, exprimée en pourcentages, inférieure à 2,5 %, de préférence inférieure à 2,2 %, de préférence inférieure à 1,7%, voire inférieure à 1,3 %.

Par ailleurs, la paroi de captation de lumière peut présenter un rapport Haze, défini comme le rapport de la réflexion diffuse sur la réflexion totale et exprimé en pourcentages, supérieur à 20 %, de préférence supérieur à 50 %, voire de préférence supérieur à 80 %.

Les faces externe et interne de la paroi de captation de lumière peuvent présenter des textures rugueuses différentes.

Par ailleurs, afin de limiter la formation d'un biofilm par accumulation du microorganisme photosynthétique sur la face externe de la paroi de captation de lumière lorsque le milieu de culture est au contact de ladite paroi, le rapport de texturation de la face externe de la paroi de captation de lumière est de préférence compris entre 40 nm et 600 nm, de préférence compris entre 80 nm et 300 nm. De plus, le pas moyen de la texture rugueuse de la face externe de la paroi de captation de lumière est de préférence inférieur à la taille du microorganisme photosynthétique. La « taille » d'un microorganisme synthétique correspond à sa plus grande dimension, et peut être mesurée par fluorimétrie. La prévention de la formation d'un biofilm opaque sur la face externe de la paroi de captation de lumière permet d'éviter que l'intensité du rayonnement dans le visible incident transmise dans la chambre de culture ne diminue progressivement de jour en jour lors de la culture du microorganisme photosynthétique.

La texture rugueuse de la face externe de la paroi de captation de lumière peut être obtenue par le procédé de gravure plasma sous vide décrit ci-dessus.

La paroi de captation de lumière peut présenter une forme d'une plaque, en particulier une forme de pavé droit.

De préférence, le filtre optique est disposé de sorte que la portion du rayonnement dans le visible transmise par le filtre optique et par la paroi de captation de lumière accède à la chambre de culture, lorsque le photobioréacteur est exposé au rayonnement dans le visible.

De préférence, la paroi de captation de lumière est disposée entre la chambre de culture et le filtre optique.

En ce qui concerne l'enceinte, elle comporte, voire peut consister en la paroi de captation de lumière.

Dans une variante, l'enceinte peut comporter une cuve, la paroi de captation de lumière définissant un couvercle de la cuve. La cuve peut être formée du même matériau que la paroi de captation de lumière.

L'enceinte peut comporter une ou plusieurs ouvertures configurées de sorte que le milieu de culture puisse circuler à travers la chambre de culture. En particulier, elle peut comporter une ouverture d'entrée et une ouverture de sortie pour le milieu de culture.

Le photobioréacteur peut en outre comporter une pompe configurée pour la circulation du milieu de culture à travers la chambre de culture. En particulier, la circulation peut être en boucle fermée, c'est-à-dire que le milieu de culture évacué de la chambre de culture à travers une ouverture de sortie est réinjecté dans la chambre de culture au travers d'une des ouvertures.

L'enceinte peut être de forme variée. Elle peut notamment présenter une forme générale parallélépipédique de révolution. Elle peut présenter la forme d'un tube creux, ouvert ou non à ses extrémités. La génératrice du tube peut suivre une courbe hélicoïdale. Le tube peut présenter des portions droites et des portions coudées, par exemple à 90° ou à 180°, séparant deux portions droites consécutives, par exemple de sorte que le tube serpente en zigzagant dans un plan.

Par ailleurs, l'enceinte définit la chambre de culture. La chambre de culture peut contenir le milieu de culture. Le milieu de culture comporte le microorganisme photosynthétique et comporte de préférence un solvant aqueux comportant des nutriments dans lequel est dispersé le microorganisme photosynthétique.

Le microorganisme photosynthétique peut être choisi parmi une bactérie photosynthétique, une cyanobactérie photosynthétique, une microalgue. Le microorganisme photosynthétique préféré est une microalgue, notamment *Phaeodactylum Tricornutum.*

De préférence, la température de transition du composé thermochrome est inférieure à la température à partir de laquelle la mort du microorganisme photosynthétique est constatée.

L'invention concerne encore un procédé de culture d'un microorganisme photosynthétique, dans lequel on expose un photobioréacteur selon l'invention à un rayonnement d'irradiation comportant des composantes dans le visible et dans l'infrarouge, la chambre de culture du photobioréacteur contenant un milieu de culture comportant un microorganisme photosynthétique.

Le rayonnement d'irradiation peut être le rayonnement solaire.

Le milieu de culture, le microorganisme photosynthétique, le rayonnement dans le visible et le rayonnement dans l'infrarouge sont de préférence tels que décrits précédemment.

### Brève description des figures

D'autres aspects de l'invention apparaitront plus clairement à la lecture de la description détaillée ci-dessous et des dessins dans lesquels :
[Fig 1] représente, de manière schématique et en coupe transversale un exemple de photobioréacteur selon l'invention;
[Fig 2] est un agrandissement d'une partie du photobioréacteur de la figure 1 ;
[Fig 3] a) à c) sont des photographies, prises en microscopie électronique à balayage, de la texture rugueuse de la face interne de la paroi de captation de lumière de différents exemples de photobioréacteurs selon l'invention.
[Fig 4] illustre la variation, en fonction de la longueur d'onde du rayonnement incident, de la transmittance optique des parois de captation de lumière représentées sur les figures 3a à 3c ; et
[Fig 5] illustre la variation, en fonction de la longueur d'onde du rayonnement incident, du rapport Haze des parois de captation de lumière représentées sur les figures 3a à 3c ;
[Fig 6] illustre la variation, en fonction de la longueur d'onde du rayonnement incident, de la réflexion totale pondérée par la réponse spectrale de l'œil des parois de captation de lumière représentées sur les figures 3a à 3c ; et
[Fig 7] illustre un autre exemple de photobioréacteur selon l'invention.

Pour des raisons de clarté, les différents éléments des figures sont représentés en échelle libre, les dimensions réelles des différentes parties n'étant pas nécessairement respectées. En particulier, les dimensions des motifs des textures rugueuses peuvent être exagérées par rapport aux dimensions des autres éléments constitutifs du photobioréacteur.

Dans la suite du texte, les expressions « compris entre ... et ... », « allant de ... à ...» et « variant de ... à ... » sont équivalentes et entendent signifier que les bornes sont incluses, sauf mention contraire.

### Description détaillée

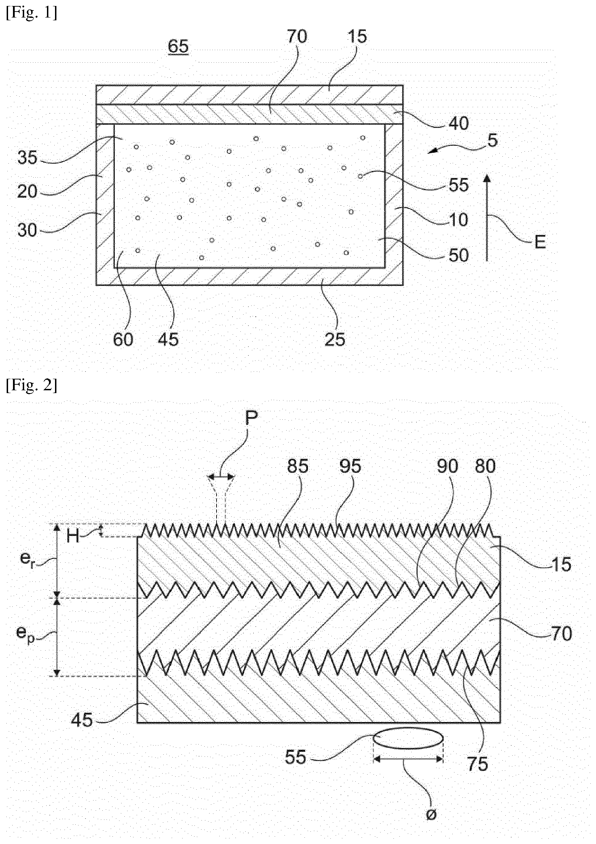

La figure 1 représente un exemple de photobioréacteur 5 selon l'invention. Le photobioréacteur comporte une enceinte 10 et un filtre optique 15 disposé au contact de l'enceinte.

L'enceinte comporte une cuve 20, présentant un fond 25 et au moins une paroi latérale 30 s'étendant à partir du fond selon une direction d'extension E. La direction d'extension peut être verticale, bien que d'autres orientations puissent être envisagées. La cuve définit une ouverture supérieure de cuve 35.

L'enceinte comporte par ailleurs un couvercle 40, sous la forme d'une plaque, disposé sur la cuve et recouvrant entièrement l'ouverture supérieure de cuve, de sorte à clore l'enceinte. La cuve et le couvercle définissent ainsi une chambre de culture 45, qui contient un milieu de culture 50 comportant le microorganisme photosynthétique 55. Le milieu de culture comporte un solvant 60 aqueux contenant des éléments essentiels à la croissance du microorganisme photosynthétique. La chambre de culture peut être hermétiquement isolée de l'extérieur 65, par exemple au moyen de joints d'étanchéité, non représentés, pris en sandwich entre la cuve et le couvercle.

Dans l'exemple de la figure 1, la paroi de captation de lumière 70 est définie par le couvercle 40. La paroi de captation de lumière est transparente à la lumière solaire et par exemple formée de verre. Les parois de fond et latérale de la cuve peuvent être en un matériau opaque à un rayonnement dans le visible. Dans une variante, la paroi de fond et la paroi latérale de fond de la cuve peuvent être des parois de captation de lumière.

La paroi de captation de lumière comporte une face externe 75 disposée en regard de la chambre de culture et une face interne 80, opposée à la face externe et séparée par l'épaisseur eₚ de la paroi de captation de lumière. Le filtre optique 15 est en contact avec et recouvre la face interne 80 de la paroi de captation de lumière. Dans une variante, le filtre optique peut recouvrir la face externe de ladite paroi.

Le filtre optique est formé par un revêtement 85 en un matériau thermochrome, par exemple en oxyde de vanadium dopé au tungstène présentant une température de transition comprise entre 38°C et 42°C. Le revêtement présente de préférence une épaisseur eᵣ comprise entre 50 nm et 800 nm. Il comporte une face interne 90 en contact de la face interne de la paroi de captation de lumière et une face externe 95 disposée à l'opposé de ladite face interne.

Par ailleurs, comme cela est illustré sur la figure 2, la face externe 95 du filtre optique et la face interne 80 de la paroi de captation de lumière présentent chacune une texture rugueuse, afin d'augmenter la transmittance optique au rayonnement dans le visible de la paroi de captation de lumière et du filtre optique. La texture rugueuse est formée par la répétition régulière d'un motif de section transverse triangulaire. La texture présente un pas P entre deux motifs et une hauteur H de motif.

En outre, la face externe 75 de la paroi de captation de lumière présente une texture rugueuse constituée elle aussi par la répétition régulière d'un motif comme la face externe du filtre optique. Cependant, le pas moyen de la texture rugueuse de la face externe de la paroi de captation de lumière est inférieur à la taille φ du microorganisme, afin de limiter la formation d'un biofilm opaque comportant le microorganisme photosynthétique sur la paroi de captation de lumière.

Le photobioréacteur peut en outre comporter des moyens, non représentés, pour renouveler le solvant aqueux et/ou alimenter le milieu de culture en nutriments, par exemple une source de potassium ou un gaz tel que du dioxyde de carbone. Il peut comporter des moyens pour extraire les produits de la croissance du microorganisme photosynthétique, par exemple un gaz tel que le dioxygène généré par l'activité photosynthétique.

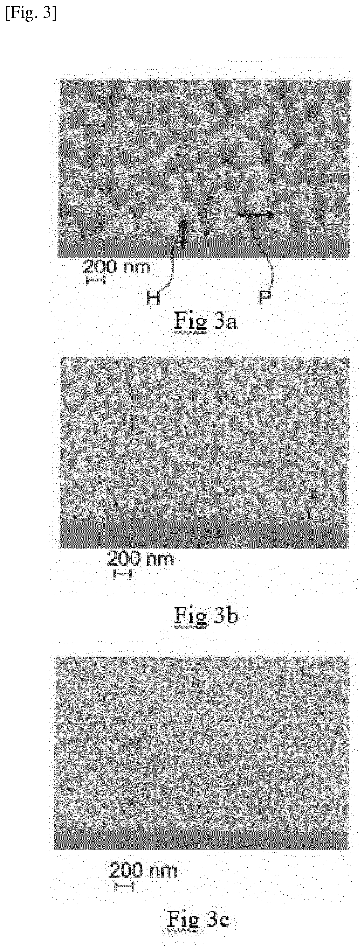

Les figures 3a à 3c sont des photographies, en microscopie à balayage, de la texture rugueuse de la face interne de la paroi de captation de lumière de trois photobioréacteurs, ci-après dénommés exemples 1 à 3. La paroi de captation de lumière illustrée sur ces figures est une plaque de verre aluminoborosilicate.

Les textures rugueuses présentées sur les figures 3a à 3c ont été obtenues par traitement plasma sous vide, de la paroi de captation de lumière, au moyen d'un équipement de gravure sèche. Le procédé de gravure sèche a été mis en œuvre au moyen d'un mélange gazeux de Trifluorométhane CHF3 et de dioxygène O2 avec un rapport CHF₃/O₂ compris entre 10 et 15, à une pression de travail comprise entre 50 mTorr et 200 mTorr, avec une densité de puissance comprise entre 1,65 W/cm² et 3,56 W/cm² (RF), et pendant une durée de traitement comprise entre 10 minutes et 30 minutes.

Comme cela peut être observé sur les figures 3a à 3c, la texture rugueuse présente une structure sensiblement régulière formée par la répétition, selon la largeur et la longueur revêtement, d'un motif ayant une forme pyramidale.

La variation des pressions de travail, densité de puissance et durée de traitement résulte en variation des pas moyen et hauteur moyenne de la texture.

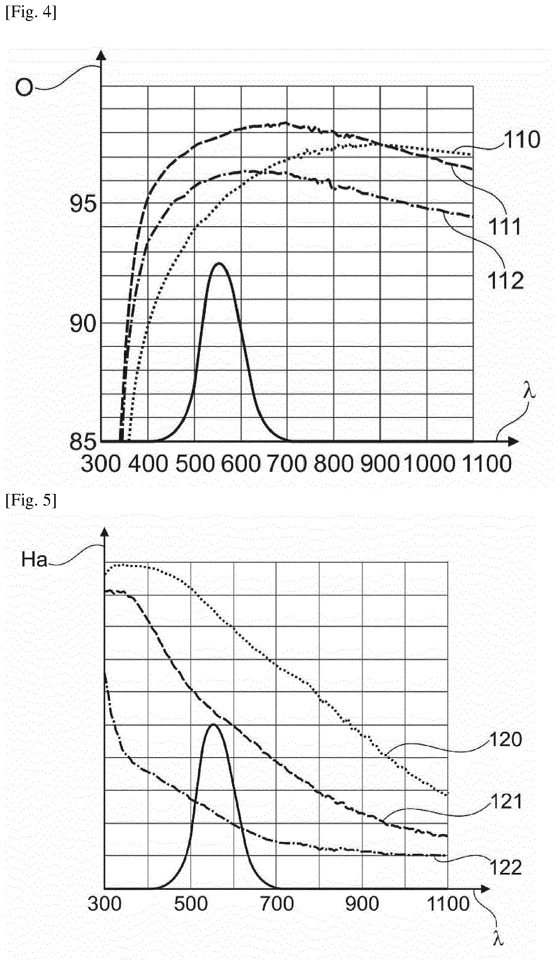

La figure 4 illustre les évolutions 110, 111 et 112 en fonction de la longueur d'onde λ, exprimée en nm, de la transmittance optique, exprimée en pourcents, des parois de captation de lumière des exemples 1 à 3 respectivement. Les parois de captation des exemples 1 à 3 sont transparentes à un rayonnement dans le visible et à un rayonnement dans l'infrarouge présentant une longueur d'onde inférieure à 1100 nm.

La figure 5 illustre les évolutions 115, 116 et 117 en fonction de la longueur d'onde λ, exprimée en nm, de la réflexion totale Rtot, exprimée en pourcents, des parois de captation de lumière des exemples 1 à 3 respectivement. La présence d'une texture rugueuse sur la face interne des parois de captation des exemples 1 à 3, bien que non essentielle à l'invention, limite la réflexion totale du rayonnement incident. La réflexion totale, illustrée sur la figure 5, est toujours inférieure à 6 % quelle que soit la longueur d'onde du rayonnement incident comprise entre 350 nm et 1100 nm pour les exemples 1 à 3. La réponse totale pondérée par la réponse spectrale de l'œil est de 2,16% au plus pour l'exemple 1, comme cela est indiqué sur le tableau 1. Elle est de l'ordre de 8% pour une paroi de captation formée du même matériau mais ne présentant pas une texture rugueuse.

La figure 5 illustre les évolutions 120, 121 et 122 en fonction de la longueur d'onde λ, exprimée en nm, du rapport Haze Ha des parois de captation de lumière des exemples 1 à 3 respectivement. Pondéré par la réponse spectrale de l'œil, le rapport Haze est d'au moins 22,0% (exemple 3). La paroi de captation de l'exemple non traité par plasma, ne présentant pas de texture rugueuse présente un rapport Haze inférieur à 1,0%.

La présence d'une texture rugueuse, bien que non essentielle à l'invention, améliore donc la transmittance optique de l'ensemble formé par le filtre optique et la paroi de captation de lumière.

### [Tableau 1]

**Tableau 1**

| Exemple | 1 | 2 | 3 | Sans traitement plasma |
|---|---|---|---|---|
| Réflexion totale (RT) pondérée par la réponse spectrale de l'œil (%) | 2,16 | 1,23 | 1,59 | 8 |
| Réflexion totale minimale sur la plage de longueur d'onde comprise entre 400 nm et 800 nm (%) | 1,28 | 1,16 | 1,11 | 8 |
| Longueur d'onde pour laquelle la réflexion totale est minimale (nm) | 785 | 610 | 330 | Non applicable |
| Réflexion diffuse (RD) pondérée par la réponse spectrale de l'œil (%) | 1,84 | 0,66 | 0,35 | <0.5 |
| Rapport Haze = RD/RT (%) | 85,2 | 53,7 | 22,0 | < 1,0 |

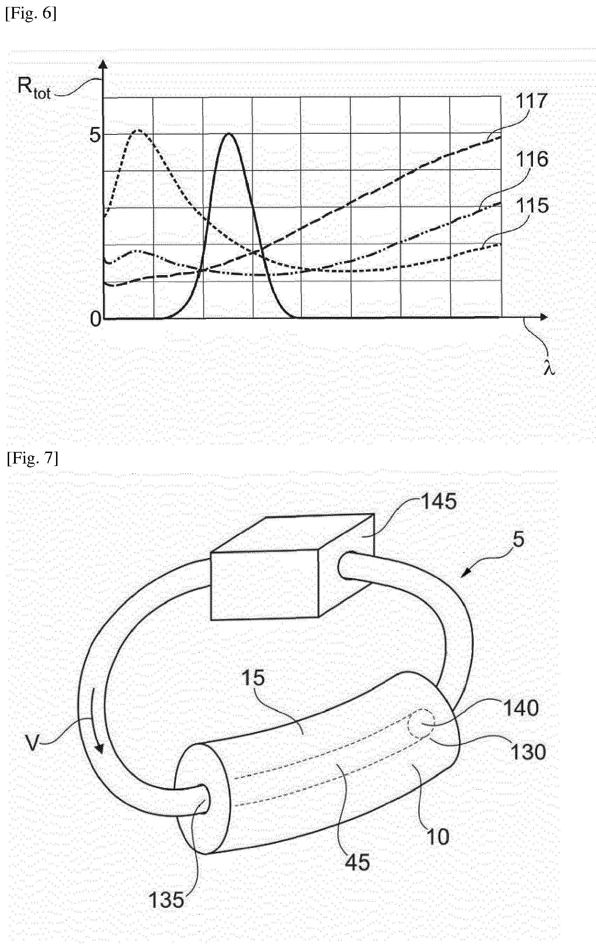

La figure 7 illustre un autre exemple de photobioréacteur selon l'invention.

Le photobioréacteur 5 de la figure 7 diffère du photobioréacteur illustré sur la figure 1 en ce que l'enceinte 10 consiste en la paroi de captation de lumière, dont la face externe 130 est définie par la face latérale extérieure du tube, et est recouverte par le filtre optique 15 sous la forme d'un revêtement formé du composé thermochrome. L'enceinte présente une forme générale tubulaire de révolution et comporte des ouvertures d'entrée 135 et de sortie 140 par lesquelles le milieu de culture 45 circule entre, respectivement sort de l'enceinte. La circulation V du milieu de culture s'effectue en boucle fermée. Une pompe 145 aspire le milieu de culture qui sort de l'enceinte par l'ouverture de sortie 140 pour le réinjecter par l'ouverture d'entrée 135.

Comme cela apparaît à la lecture de la description, le photobioréacteur selon l'invention assure une régulation passive de la température du milieu de culture, favorisant ainsi la culture, à faible coût avec un rendement élevé du microorganisme photo synthétique.

Bien entendu, l'invention n'est pas limitée aux exemples de modes de réalisation du dispositif et de mise en œuvre du procédé décrits ci-dessus.

## Revendications

1. Photobioréacteur (5) pour la culture d'un microorganisme photosynthétique (55), le photobioréacteur comportant :
- une enceinte (10) comportant une paroi de captation de lumière (70) et définissant une chambre de culture (45) pour contenir un milieu de culture (50) contenant au moins le microorganisme photosynthétique, la paroi de captation de lumière étant transparente à un rayonnement dans l'infrarouge et à un rayonnement dans le visible, et
- un filtre optique (15) pour filtrer un rayonnement d'irradiation dirigé vers la chambre de culture,
le filtre optique étant transparent au rayonnement dans le visible et contenant au moins un composé thermochrome,
le filtre optique étant transparent au rayonnement dans l'infrarouge à une température d'au moins 10°C inférieure à la température de transition du composé thermochrome et présentant une transmittance optique au rayonnement dans l'infrarouge inférieure ou égale à 20 % à une température d'au moins 10°C supérieure à la température de transition du composé thermochrome.

2. Photobioréacteur selon la revendication 1, dans lequel la température de transition est supérieure ou égale à 30 °C, de préférence supérieure ou égale à 35 °C et/ou inférieure ou égale à 55 °C, et de préférence inférieure ou égale à 50 °C, voire même inférieure ou égale à 45 °C.

3. Photobioréacteur selon l'une quelconque des revendications 1 et 2, dans lequel le composé thermochrome est un oxyde thermochrome, optionnellement dopé, choisi dans le groupe formé par VO₂, BiVO₄, NbO₂ et leurs mélanges, de préférence est l'oxyde de vanadium, dopé au tungstène VO₂:W.

4. Photobioréacteur selon l'une quelconque des revendications précédentes, dans lequel le filtre optique présente une transmittance optique au rayonnement dans l'infrarouge supérieure ou égale à 80 %, voire supérieure ou égale à 90 %, respectivement inférieure ou égale à 20 %, voire inférieure ou égale à 10 %, à une température d'au moins 5 °C inférieure, respectivement d'au moins 5 °C supérieure à la température de transition du composé thermochrome.

5. Photobioréacteur selon l'une quelconque des revendications précédentes, dans lequel le filtre optique est disposé sur la paroi de captation de lumière.

6. Photobioréacteur selon l'une quelconque des revendications précédentes, dans lequel le filtre optique est formé par un revêtement (85) recouvrant au moins partiellement, voire totalement une face de la paroi de captation de lumière.

7. Photobioréacteur selon la revendication précédente, dans lequel le revêtement présente des faces externe (95) et interne (90), la face interne étant au contact de la paroi de captation de lumière, la face externe étant opposée à la face interne et présentant une texture rugueuse sous la forme d'un réseau formé par une succession régulière d'un motif en relief.

8. Photobioréacteur selon l'une quelconque des revendications précédentes, dans lequel le filtre optique est disposé de sorte que la portion dans le visible du rayonnement d'irradiation transmis par le filtre optique et par la paroi de captation de lumière accède à la chambre de culture, lorsque le photobioréacteur est exposé au rayonnement dans le visible.

9. Photobioréacteur selon l'une quelconque des revendications précédentes, dans lequel la paroi de captation de lumière est disposée entre la chambre de culture et le filtre optique.

10. Photobioréacteur selon l'une quelconque des revendications précédentes, dans lequel la paroi de captation de lumière comporte une face externe (75) en regard de la chambre de culture et une face interne (80) opposée à la face externe et au contact du filtre optique, la face externe et/ou la face interne de la paroi de captation de lumière présentant une texture rugueuse, de préférence présentant la forme d'un réseau formé par une succession régulière d'un motif en relief.

11. Photobioréacteur selon la revendication précédente, dans lequel le rapport de texturation de la face interne de la paroi de captation de lumière est compris entre 1,30 et 2,00, de préférence comprise entre 1,50 et 1,90.

12. Photobioréacteur selon l'une quelconque des revendications précédentes, dans lequel la chambre de culture contient le milieu de culture, le microorganisme photosynthétique étant choisi parmi une bactérie photosynthétique, une cyanobactérie photosynthétique, une microalgue notamment eucaryote.

13. Photobioréacteur selon la revendication précédente, dans lequel la face externe de la paroi de captation de lumière comporte une texture rugueuse dont le pas moyen est inférieur à la taille (Φ) du microorganisme photosynthétique.

14. Procédé de culture d'un microorganisme photosynthétique, dans lequel on expose un photobioréacteur selon l'une quelconque des revendications précédentes à un rayonnement d'irradiation comportant des composantes dans le visible et dans l'infrarouge, la chambre de culture du photobioréacteur contenant un milieu de culture comportant un microorganisme photosynthétique.

15. Procédé selon la revendication précédente, dans lequel le rayonnement d'irradiation est le rayonnement solaire.
